Europäisches Patentamt

European Patent Office

Office européen des brevets

(19)

(11) Publication number: **0 204 284**

A2

# (12) EUROPEAN PATENT APPLICATION

(21) Application number: **86107417.7**

(22) Date of filing: **02.06.86**

(51) Int. Cl.⁴: **C 12 N 9/20**
//(C12N9/00, C12R1:38)

(30) Priority: **05.06.85 JP 120400/85**

(43) Date of publication of application:
**10.12.86 Bulletin 86/50**

(84) Designated Contracting States:
**BE DE FR GB NL SE**

(71) Applicant: **SAPPORO BREWERIES LIMITED**
**No. 10-1, Ginza 7-chome Chuo-ku**
**Tokyo(JP)**

(72) Inventor: **Nishio, Toshiyuki**
**No. 514-2, Kogawa**
**Yaizu-shi Shizuoka-ken(JP)**

(72) Inventor: **Chikano, Takahide**
**No. 1098-1, Nakada**
**Shizuoka-shi Shizuoka-ken(JP)**

(72) Inventor: **Kamimura, Minoru**
**No. 514-2, Kogawa**
**Yaizu-shi Shizuoka-ken(JP)**

(74) Representative: **Türk, Gille, Hrabal**
**Bruckner Strasse 20**
**D-4000 Düsseldorf 13(DE)**

(54) A novel lipase.

(57) The lipase is a novel microbiological enzymatic product produced by *Ps. fragi* 22.39B and has relatively high activity in an alkaline condition with an optimum pH of 9.0 and an optimum temperature of 65 to 70°C. The lipase is active for the decomposition of not only fatty acid triglycerides, oils and fats, but also low-molecular monoesters such as methyl oleate and butyl oleate as well as water-soluble esters such as polyoxyethylene sorbitan fatty acid esters.

*FIG.1*

o --- Citric acid-Sodium citrate buffer
• --- Imidazole-HCl buffer
□ --- Tris-HCl buffer
■ --- Sodium carbonate-Sodium
hydrogencarbonate buffer

EP 0 204 284 A2

Croydon Printing Company Ltd.

BACKGROUND OF THE INVENTION

The present invention relates to a novel lipase having excellent heat resistance and an optimum pH at a high alkalinity as well as high activity on low-molecular monoesters and water-soluble esters.

Lipase is a class of enzymes highlighted in recent years due to their usefulness in various applications as a digestive and a reagent for the determination of lipids in blood in therapy or medical science, as an enzyme for the decomposition of oils and fats in the manufacture of soaps and as an additive in detergents. A large number of enzymes are known to belong to the class of lipase and each of them has its own advantageous and disadvantageous properties so that it is desirable to develop a lipase enzyme having higher activity and larger versatility in many applications in respect of, for example, heat resistance and optimum pH. A lipase capable of withstanding a high temperature and having activity at a high alkalinity would be useful as an additive in alkaline detergents or in the decomposition of oils and fats at a high temperature.

The inventors have previously proposed a method for obtaining an alkaline lipase by culturing bacteria of Pseudomonas fragi belonging to the genus of Pseudomonas (see Japanese Patent Publication 56-28517). The novel lipase of the present invention can be isolated in electrophoretically homogeneous form from the above mentioned enzyme product and is characterized by the chemical and physical properties described below.

- 1 -

Thus, the chemical and physical properties, by which the inventive novel lipase is characterized, are as described below. The activity of the enzyme has been determined by the PVA emulsion method proposed by Yamada, et al. and described in Journal of the Agricultural Chemical Society of Japan, volume 36, page 860 (1962) with some modifications if not mentioned otherwise, taking an amount of the enzyme liberating a micro-equivalent of a fatty acid for 1 minute as one unit (U) (see Japanese Patent Publication 56-28517).

(a) Substrate specificity

The enzyme is highly active for the decomposition of various kinds of oils and fats occurring in nature as well as triolein, tributyrin, triacetin and the like. The enzyme also has good activity for the decomposition of monoesters such as methyl oleate, butyl oleate and the like and water-soluble esters such as polyoxyethylene sorbitan fatty acid esters and the like.

(b) Optimum pH

When the substrate is an aqueous emulsion of olive oil, the optimum pH of the enzyme is 9.0. Thus, the enzyme has an optimum pH at a relatively high alkalinity.

(c) Optimum temperature

The optimum temperature of the enzyme is 65 to 70°C for an aqueous emulsion of olive oil as the substrate.

(d) pH stability

The enzyme is stable at 4°C with a pH of 4.0 or higher, at 30°C with a pH of 6.5 or higher and at 50°C with a pH of 8.0 or higher.

(e) Thermal stability

The enzyme is stable for 24 hours with a pH of 9.0 at a temperature up to 51°C but gradually deactivated at 51°C or higher.

(f) Activation and deactivation

The enzyme is deactivated by $Zn^{2+}$, $Fe^{2+}$ and $Fe^{3+}$, in particular, among metal ions and also deactivated by cationic surface active agents such as cetyl trimethyl ammonium bromide and the like. The activity of the enzyme is somewhat increased by sodium deoxycholate, sodium taurocholate and Triton X-100.

(g) Isoelectric point

The isoelectric point of the enzyme is at about pI 7 as determined by the method of isoelectric focusing using Ampholite as the carrier.

(h) Molecular weight

The molecular weight of the enzyme is about 33,000 as determined by the method of SDS-electrophoresis according to the procedure proposed by Weber and Osborn, and about 32,500 as determined by the method of gel filtration using Sephadex G-100.

(i) Derivation

The enzyme is obtained from _Pseudomonas_ _fragi_ 22.39B, of which a strain is deposited at Fermentation Research Institute, Agency of Industrial Science and Technology, Japan, as FERM P-1339 (FERM BP-1051).

- 3 -

## BRIEF DESCRIPTION OF THE DRAWING

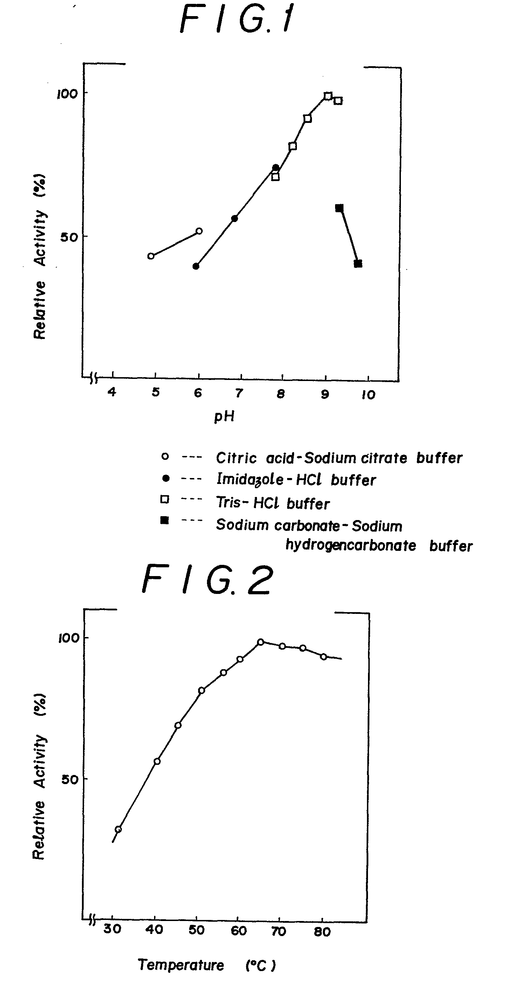

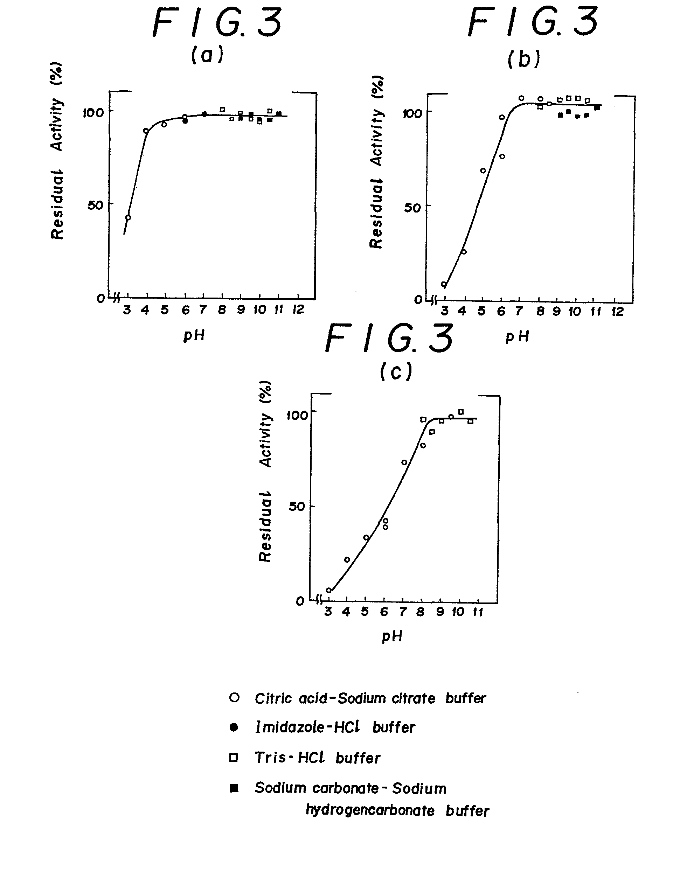

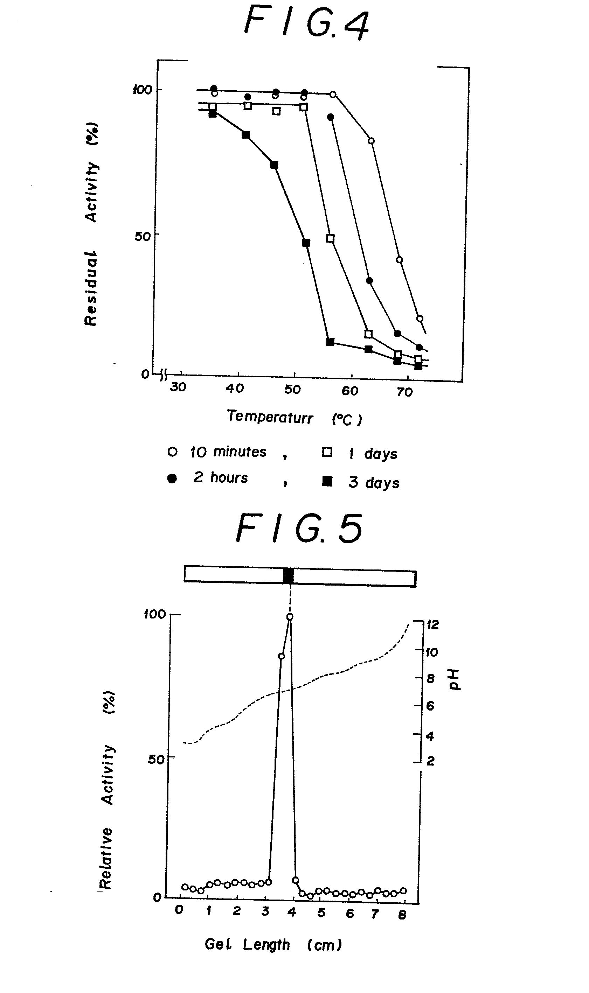

FIGURE 1 is a graph showing the activity of the enzyme as a function of pH, FIGURE 2 is a graph showing the activity of the enzyme as a function of temperature, FIGURES 3a, 3b and 3c are each a graph showing the pH stability of the enzyme and FIGURE 4 is a graph showing the thermal stability of the enzyme.

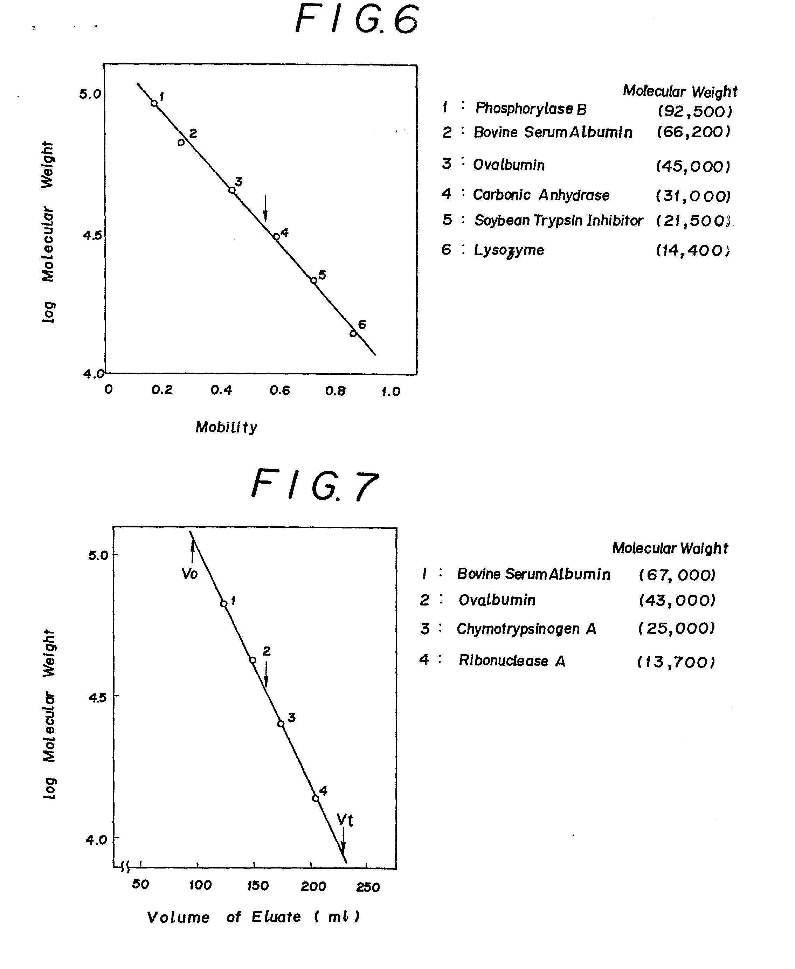

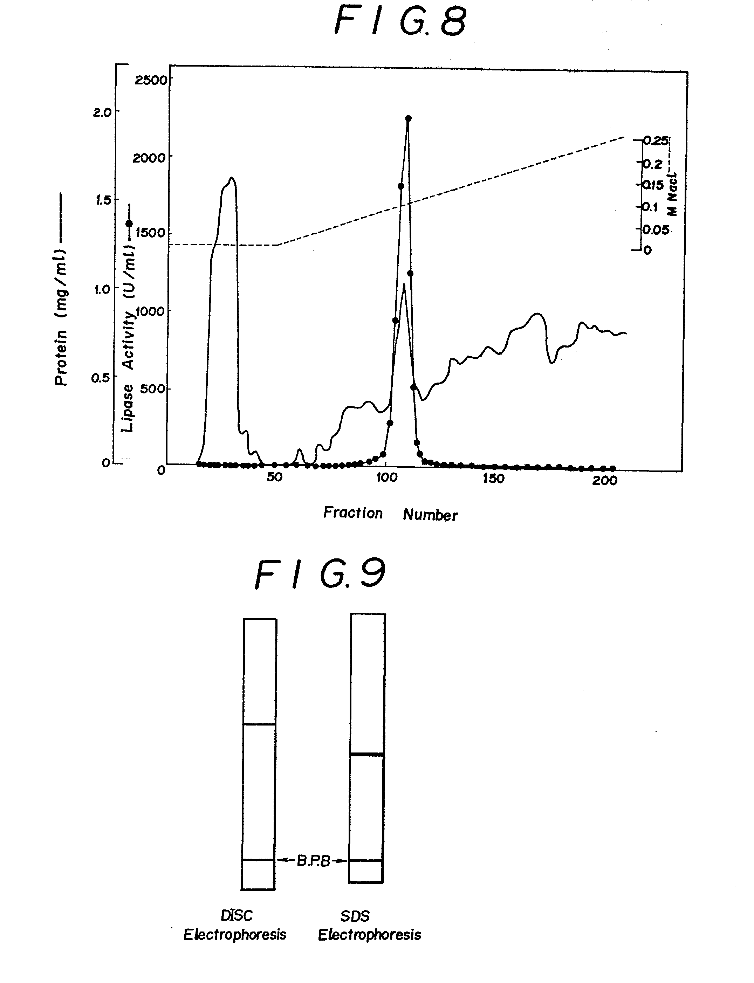

FIGURE 5 is a graph showing the isoelectric point of the enzyme, FIGURE 6 is a diagram obtained in the SDS electrophoresis for the molecular weight determination of the enzyme, FIGURE 7 is a diagram obtained in the gel filtration for the molecular weight determination of the enzyme, FIGURE 8 is a chromatogram obtained in the purification procedure of the enzyme and FIGURE 9 is an electrophoretic diagrams of the enzyme.

## DETAILED DESCRIPTION OF THE PREFERRED EMBODIMENTS

The inventive lipase is not limited by the species of the microorganism which produces the enzyme provided that the microorganism is a bacterium belonging to the genus of Pseudomonas and capable of producing an enzyme having the above described characteristic chemical and physical properties. A particularly preferable microorganism is the above mentioned Ps. fragi.

The culturing conditions of the microorganism producing the novel lipase of the invention are not particularly limitative and should be determined so as to ensure highest productivity of the desired enzyme. A typical procedure of the culturing method is disclosed in the Example of Japanese Patent Publication 56-28517.

After completion of culturing of the microorganism, the inventive lipase is purified by a suitable known method of, for example, a chromatography including ion exchange, gel filtration and the like. Following is a detailed description of the thus purified inventive novel lipase.

I. Chemical properties of the enzyme

1. Substrate specificity

The enzyme is active for the decomposition of the substrate, such as various kinds of esters and naturally occurring oils and fats. Tables 1-1 and 1-2 given below give the relative rate of decomposition of esters (Table 1-1) and naturally occurring oils and fats (Table 1-2) in % taking triolein and olive oil, respectively, as the reference.

Table 1-1

| Ester as the substrate | Relative rate of decomposition, % |
|---|---|
| Methyl oleate | 87 |
| Ethyl oleate | 59 |
| Butyl oleate | 95 |
| Methyl butyrate | 23 |
| Sorbitan monolaurate (Span 20) | 66 |
| Sorbitan monooleate (Span 80) | 36 |
| Polyoxyethylene sorbitan monolaurate (Tween 20) | 96 |
| Polyoxyethylene sorbitan monooleate (Tween 80) | 50 |
| Triacetin | 108 |
| Tributyrin | 243 |
| Triolein | 100 |

Table 1-2

| Oil or fat as the substrate | Relative rate of decomposition, % |
| --- | --- |
| Olive oil | 100 |
| Corn oil | 109 |
| Soybean oil | 107 |
| Rapeseed oil | 99 |
| Linseed oil | 101 |
| Peanut Oil | 99 |
| Cottonseed oil | 113 |
| Coconut oil | 116 |
| Beaf tallow | 94 |
| Whale oil | 72 |

To summarize the above given results, the lipase of the invention has an activity for the decomposition of not only triglycerides as a matter of course but also low-molecular monoesters exhibiting a good activity also as an esterase. The lipase also has an activity for the decomposition of water-soluble polyoxyethylene sorbitan fatty acid esters such as the Tween-type surface active agents. It is concluded accordingly that the lipase is active for the decomposition of carboxylate esters in general regardless of the solubility or insolubility thereof in water including glycerol fatty acid esters only as a part of the substrates. The kind of animals and plants also has little influence on the activity of the enzyme for the decomposition of naturally occurring oils and fats of animal or vegetable origin.

Different from the above mentioned standard procedure of the PVA emulsion method, the results tabulated in Tables 1-1 and 1-2 were obtained by the shaking method according to the conditions and procedures described below.

(a) Formulation of the solution for the reaction

| | |
|---|---|
| Substrate | 0.2 g |
| 0.2M Tris-HCl buffer solution (pH 9.0) | 4 ml |
| 60 mM Calcium chloride solution | 1 ml |
| Enzyme solution | 1 ml |

(b) Procedure

The solution described above was taken in an Erlenmeyer flask of 100 ml capacity and shaken for 30 minutes in a thermostat at 37°C at a rate of 90 rpm along an 8-shaped line. After completion of the above mentioned reaction time, 20 ml of a 1:1 mixture of acetone and ethyl alcohol were added to the flask and the resultant solution was titrated within a length of time with a 0.05N aqueous solution of sodium hydroxide using phenol phthalein as the indicator.

2. Optimum pH

Determination of the optimum pH was undertaken using an aqueous emulsion of olive oil as the substrate. The buffer solutions used for the control of the pH of the solution for the reaction included citric acid-sodium citrate buffer solutions having a pH of 5 to 6, imidazole-hydrochloric acid buffer solutions having a pH of 6 to 8, tris-hydrochloric acid buffer solutions having a pH of 8 to 10 and sodium carbonate-sodium hydrogen carbonate buffer solutions having a pH of 9.5 to 10.5.

The results were as shown in FIGURE 1, from which it was concluded that the optimum pH of the enzyme was 9.0 when the substrate was the aqueous emulsion of olive oil.

3. Optimum temperature

Determination of the optimum temperature was undertaken using an aqueous emulsion of olive oil as the substrate with a controlled pH of 9.0. The temperature of determination was 35°C or higher increased stepwise with about 5°C intervals. The results are shown in FIGURE 2, from which it was concluded that the activity of the enzyme was highest at a temperature of 65 to 70°C for the decomposition of olive oil in aqueous emulsion as the substrate.

4. pH stability

The buffer solutions used for the test of the pH stability of the enzyme included citric acid-sodium citrate buffer solutions having a pH of 3 to 6, imidazole-hydrochloric acid buffer solutions having a pH of 6 to 8, tris-hydrochloric acid buffer solutions having a pH of 8 to 10.5 and sodium carbonate-sodium hydrogencarbonate buffer solutions having a pH of 9 to 11. The pH stability was tested by keeping a solution prepared by adding the purified enzyme into these buffer solutions in a concentration of 200 U/ml or about 100 µg/ml for 24 hours at a temperature of (a) 4°C, (b) 30°C or (c) 50°C and determining the residual activity of the enzyme to give the results shown in FIGURES 3a, 3b and 3c, respectively. As is understood from these results, the enzyme is stable at low temperatures over a wide range of pH from an acidity of pH 4.0 to alkalinity. The enzyme is also stable even at a relatively high temperature when the ph is within a range of neutrality to alkalinity.

5. Thermal stability

Thermal stability of the enzyme was tested by adding the purified enzyme to a 10 mM THB in a concentration of 200 U/ml or about 100 $\mu$g/ml and, while the solution was kept standing at a temperature of 35 to 72°C for a length of time of up to 3 days, by periodically determining the residual activity of the enzyme in the solution. The results are shown in FIGURE 4, from which it is understood that the enzyme was stable at a temperature lower than 51°C for at least 24 hours but gradually deactivated thereafter in the lapse of time. Deactivation of the enzyme at 51°C was not so rapid that almost about 50% of the residual activity is left even after 3 days at the temperature. When the temperature exceeded 56°C, however, the enzyme was deactivated at a relatively high velocity so that it could be concluded that the practical upper limit of the temperature for the thermal stability of the enzyme was 50 to 55°C.

6. Activation and deactivation

The influence of metal ions on the enzymatic actvity was examined by keeping a 9:1 by volume mixture of a 5U/ml solution of the enzyme and a 10 mM solution of the metal ions at 25°C for 30 minutes followed by the determination of the residual activity of the enzyme in the solution. No calcium chloride was contained in the reaction mixture. The results are shown in Table 2, from which it is understood that the ions of $Zn^{2+}$, $Fe^{2+}$ and $Fe^{3+}$ had a deactivating effect on the enzyme.

- 9 -

Table 2

| Metal salt | Residual activity, % |
|---|---|
| $MgCl_2$ | 97.2 |
| $CaCl_2$ | 105.5 |
| $SrCl_2$ | 92.7 |
| $BaCl_2$ | 92.7 |
| $AlCl_3$ | 93.6 |
| $SnCl_2$ | 79.8 |
| $PbCl_2$ | 78.0 |
| $CuCl_2$ | 90.8 |
| $AgCl$ | 93.6 |
| $ZnCl_2$ | 34.9 |
| $HgCl_2$ | 83.5 |
| $MnCl_2$ | 81.7 |
| $FeCl_2$ | 36.7 |
| $FeCl_3$ | 33.0 |
| $CoCl_2$ | 86.6 |
| $NiCl_2$ | 98.9 |
| $NaCl$ | 98.0 |
| $KCl$ | 98.0 |
| $LiCl$ | 97.5 |

Similarly, the influence of surface active agents on the enzymatic activity was examined by keeping a 9:1 by volume mixture of a 10 U/ml solution of the enzyme and a 4% aqueous solution of the surface active agent at 25°C for 30 minutes followed by the determination of the residual activity of the enzyme in the solution. The results are shown in Table 3, from which it is understood that the cationic surface active agents tested generally had a deactivating effect on the enzyme while the non-ionic and anionic surface active agents included those having a deactivating effect and those having an activating effect.

- 10 -

Table 3

0204284

| Surface active agent | | Residual activity, % |
|---|---|---|
| Cationic | Dodecyl trimethyl ammonium chloride | 35 |
| | Cetyl trimethyl ammonium bromide | 12 |
| | n-Dodecyl trimethyl ammonium bromide | 46 |
| Anionic | Sodium dodecylsulfate | 63 |
| | Sodium cholate | 104 |
| | Sodium deoxycholate | 115 |
| | Sodium taurocholate | 114 |
| Non-ionic | Polyoxyethylene (10) octyl phenyl ether (Triton X-100) | 105 |
| | Polyoxyethylene sorbitan monooleate (Tween 80) | 115 |
| | Polyoxyethylene (10) oleyl ether (Brij 96) | 55 |
| | Sorbitan monooleate (Span 80) | 56 |

II. Physical properties of the enzyme

1. Isoelectric point (pI)

The isoelectric point of the enzyme was determined by the method of isoelectric focusing using Ampholite having a pH of 3.5 to 10 as the carrier. FIGURE 5 of the accompanying drawing shows the results of the electrophoresis carried out first at a constant voltage of 100 volts for 2 hours and then at a constant voltage of 200 volts for 5 hours, from which a value of pI = 6.9 was obtained.

2. Molecular weight

FIGURE 6 shows the results of the SDS electrophoresis undertaken according to the method of Weber and Osborn, from which a molecular weight of about 33,000 is obtained.

- 11 -

The molecular weight markers used in this case included lysozyme having a molecular weight of 14,400, soybean trypsin inhibitor having a molecular weight of 21,500, carbonic anhydrase having a molecular weight of 31,000, ovalbumin having a molecular weight of 45,000, bovine serum albumin having a molecular weight of 66,200 and phosphorylase B having a molecular weight of 92,500. The dyed band of the enzyme under testing appeared between those of the carbonic anhydrase and ovalbumin and the molecular weight of the enzyme could be calculated from the gradient of the straight line giving the mobility as a function of molecular weight.

Separately, the molecular weight of the enzyme was determined also by the method of gel filtration using a column having an inner diameter of 1.8 cm and a length of 90 cm filled with Sephadex G-100 after full equilibration with a 10 mM THB containing 0.2M of sodium chloride to give the results shown in FIGURE 7, from which a molecular weight of about 32,500 was obtained. The molecular weight markers used in the gel filtration included ribonuclease A having a molecular weight of 13,700, chymotrypsinogen A having a molecular weight of 25,000 ovalbumin having a molecular weight of 43,000 and bovine serum albumin having a molecular weight of 67,000. The enzyme was eluted between the chymotripsinogen A and ovalbumin and the molecular weight of the enzyme was calculated from the gradient of the straight line giving the eluate volume as a function of molecular weight. The good coincidence of the values obtained by the two different methods supported the conclusion that the molecular weight of the enzyme was about 33,000.

As is described in detail, the novel lipase of the invention also has a nature of esterase with a relatively high optimum temperature of 65 to 70°C and high thermal stability. The enzyme is very stable at neutrality to alkalinity having a high optimum pH. The enzyme has a high specific activity and is quite stable not only in a dry state but also in the form of an aqueous solution. Therefore, the enzyme is promisingly useful in various fields of applications as a reagent for clinical examination, as a decomposing agent of oils and fats, as an additive in detergents and so on.

As is known, several lipases have been reported as the products of bacterial species belonging to the genus of Pseudomonas including Ps. fluorescens, Ps. mefitica var. lipolytica, Ps. aeruginosa and Ps. fragi NRRL B-25. The lipase of the present invention can be distinguished from these known lipases without ambiguity in view of the comparison of the properties summarized below.

A. Bacterial species: Ps. fragi 22·39B

(1) Literature

Specification of the present invention

(2) Substrate specificity

Active for the decomposition of triglycerides including tributyrin, triacetin and triolein, monoesters including methyl oleate and butyl oleate, and polyoxyethylene sorbitan fatty acid esters

(3) Optimum pH

pH 9.0 for olive oil in an aqueous emulsion as the substrate

- 13 -

(4) Optimum temperature

65 to 70°C for olive oil in an aqueous emulsion as the substrate

(5) Stability

Stable at 4°C with a pH of 4.0 or higher, at 30°C with a pH of 6.5 or higher and at 50°C with a pH of 8.0 or higher, stable for 24 hours at 50°C at a pH of 9.0

(6) Molecular weight

33,000 by the SDS electrophoresis and 32,500 by the gel filtration

(7) Activation and deactivation

Deactivated by zinc chloride, iron (II) chloride and iron (III) chloride but not inhibited by sodium taurocholate

B. Bacterial species: Ps. fluorescens

(1) Literature

Biochim. Biophys. Acta, volume 488, pp. 353-358 (1977)

(2) Substrate specificity

Active for triglycerides including tricaproin and tri-palmitin

(3) Optimum pH

pH 7.0 for sesame oil in an aqueous emulsion as the substrate

(4) Optimum temperature

50 to 55°C for sesame oil in an aqueous emulsion as the substrate

(5) Stability

Stable for 30 minutes at 40°C with a pH of 7.0, and at least 80% of activity retained after 60 minutes at 37°C with a pH of 5.0 to 11.0

- 14 -

6) Molecular weight

32,000 by the gel filtration

(7) Activation and deactivation

Almost no deactivation by iron (III) chloride and mercury (II) chloride

C. Bacterial species: Ps. mefitica var. lipolytica

(1) Literatures

J. Ferment. Technol., volume 49, p. 128 (1971); and Japanese Patent Publication 50-25553

(2) Substrate specificity

Active for triglycerides including tributyrin and trimyristin but little active for monoesters such as methyl butyrate and polyoxyethylene sorbitan fatty acid esters

(3) Optimum pH

pH 7.0 for olive oil in an aqueous emulsion as the substrate

(4) Optimum temperature

70°C for olive oil in an aqueous emulsion as the substrate

(5) Stability

Stable at 37°C but 25% deactivation at 73°C after 20 minutes with a pH of 3 to 11.5

(6) Molecular weight

110,000 by the gel filtration

(7) Activation and deactivation

Inhibited by mercury (II) chloride, copper sulfate and iron (III) chloride

D. Bacterial species: Ps. aeruginosa

(1) Literature

Biochim. Biophys. Acta, volume 206, pp. 380-391 (1970)

(2) Substrate specificity

Active for triglycerides including tributyrin

(3) Optimum pH

pH 8.9 for eddiol in an aqueous emulsion as the substrate

(4) Optimum temperature

40°C for coconut oil in an aqueous emulsion as the substrate

(5) Stability

50% activity retained after 50 minutes at 50°C but completely deactivated after 20 minutes at 60°C

(6) Molecular weight

Not described

(7) Activation and deactivation

Inhibited by protamine and sodium taurocholate

E. Bacterial species: _Ps. fragi_ NRRL B-25

(1) Literature

J. Gen. Microbiol., volume 48, pp. 317-328 (1967)

(2) Substrate specificity

Active for triglycerides including tributyrin and triolein but inactive for monoesters such as methyl butyrate and methyl oleate

(3) Optimum pH

pH 8.6-8.7 for tributyrin in an aqueous emulsion as the substrate

(4) Optimum temperature

Not described

(5) Stability

Completely deactivated after 10 minutes at 40°C, 50% activity retained after 10 minutes at 35°C, and stable at 2°C with a pH of 6 to 7.8

(6) Molecular weight

Not described

(7) Activation and deactivation

Not described


In the following, the novel lipase of the invention is described in more detail by way of an example.


EXAMPLE

(1) Cultivation of bacteria and preparation of enzyme solution

A strain of Ps. fragi 22·39B (FERM P-1339, FERM BP-1051) belonging to the genus of Pseudomonas was cultured under aeration for 2 days at 27°C in a culture medium in a jar of 150 liter capacity having a controlled pH of 7.0 and containing 1% of beaf tallow, 2% of soybean flour, 1% of corn steep liquor, 0.4% of peptone, 0.1% of ammonium sulfate, 0.05% of magnesium sulfate, 0.5% of calcium carbonate and 0.1% of dipotassium phosphate.

After completion of culturing, the cultured broth was subjected to centrifugal separation at a velocity of 8000 rpm for 20 minutes to collect the supernatant.

(2) Purification of enzyme

The clear liquid obtained as the supernatant in the centrifugal separation was acidified by adding hydrochloric acid to have a pH of 4.0 so that precipitates were formed in the solution and the precipitates were collected by centrifuge at 8000 rpm for 20 minutes. This procedure was carried out at 4°C.

The thus collected precipitates were dissolved in a 10 mM tris-hydrochloric acid buffer solution having a pH of 9.0, referred to as THB hereinbelow, in a volume of one tenth of the supernatant as obtained by the centrifugal separation of the cultured broth and the solution was subjected to the fractionation with ammonium sulfate in the manner as follows. Thus, ammonium sulfate was added to the solution in a concentration of 20% saturation to precipitate a part of the dissolved substances in the solution other than the lipase and the precipitates were removed from the solution by centrifugation at 10,000 rpm for 20 minutes. Thereafter, an additional amount of ammonium sulfate was added to the solution to give a concentration of 40% saturation so that the lipase contained in the solution was precipitated. The precipitates of the lipase collected by centrifugation at 10,000 rpm for 20 minutes were then dissolved in a small volume of a 10 mM THB to give a crude enzyme solution which was taken in a tube for dialysis and subjected to thorough dialysis against a 10 mM THB.

After completion of the dialysis, the crude enzyme solution was adsorbed on DEAE-Toyopearl 650M gel in a chromatographic column after full equilibration with a 10 mM THB. After washing of the gel with a 10 mM THB to completely remove the unadsorbed materials, elution was performed by use of a 10 mM THB containing 0.5M of sodium chloride as the eluant. The eluate was collected and subjected to full dialysis against a 10 mM THB.

After completion of the dialysis, the above mentioned crude enzyme solution was adsorbed on DEAE-Sephalose CL-6B gel contained in a chromatographic column having an inner diameter of 3.6 cm and a length of 16 cm after thorough equilibration with a 10 mM THB containing 1% of Triton X-100, referred to as THB·TX100 hereinbelow. After washing of the gel with the 10 mM THB·TX100 to completely remove the unadsorbed materials, elution was performed by use of the same buffer solution as such and the same buffer solution further containing 0.25M of sodium chloride in such a manner as to produce a concentration gradient along the column. The velocity of elution was 30 ml/hour and the eluate was fractionated into fractions of each 10 ml volume. FIGURE 8 illustrates the chromatogram obtained in this elution.

FIGURE 9 illustrates the electrophoretic diagrams of the final product prepared by the above described purification procedure obtained by the disc electrophoresis at a pH of 9.5 using a 7% acrylamide gel and by the SDS electrophoresis using a 7.5% acrylamide gel. Table 4 below shows the activity in units U, specific activity in U/mg protein and yield in % of the intermediate or product after each step of the purification.

Table 4

| Step of purification | Activity, U | Specific activity, U/mg protein | Yield, % |
|---|---|---|---|
| Filtrate of cultured broth | 305,000 | 31.8 | 100 |
| Solution of precipitates by acidification | 300,000 | 78.6 | 98.3 |
| Salting-out with ammonium sulfate by 20-40% saturation | 267,200 | 106 | 87.2 |
| Chromatography on DEAE-Toyopearl 650M | 190,500 | 120 | 62.5 |
| Chromatography on DEAE-Sephalose CL-6B | 145,200 | 2145 | 47.6 |

WHAT IS CLAIMED IS:

1. A lipase which is characterized by the properties including;

(a) a substrate specificity: the enzyme is active for the decomposition of fatty acid triglycerides, oils and fats, low-molecular monoesters and water-soluble esters;

(b) an optimum pH: pH 9.0 when the substrate is olive oil in an aqueous emulsion;

(c) an optimum temperature: in the range from 65 to 70°C when the substrate is olive oil in an aqueous emulsion; and

(d) a thermal stability: the enzyme is stable for 24 hours at a temperature lower than 51°C with a pH of 9.0.

2. The lipase as claimed in claim 1 which is further characterized by the behavior of deactivation that the enzyme is deactivated by the metal ions selected from the group consisting of $Zn^{2+}$, $Fe^{2+}$ and $Fe^{3+}$ and by a cationic surface active agent.

3. The lipase as claimed in claim 1 which is further characterized by an isoelectric point (pI) of about 7 as determined by the method of isoelectric focusing.

4. The lipase as claimed in claim 1 which is further characterized by a molecular weight of about 33,000.

5. The lipase as claimed in claim 1 which is a microbiological product produced by Pseudomonas fragi 22.39B (FERM BP-1051).

6. Use of Pseudomonas fragi 22.39B (FERM BP-1051) for preparing the lipase as claimed in any of claims 1 to 4.

1/5

# F I G. 1

Relative Activity (%)

pH

○ --- Citric acid-Sodium citrate buffer
● --- Imidazole-HCl buffer
□ --- Tris-HCl buffer
■ --- Sodium carbonate-Sodium
       hydrogencarbonate buffer

# F I G. 2

Relative Activity (%)

Temperature (°C)

# F I G. 3
## (a)

Residual Activity (%) vs pH

# F I G. 3
## (b)

Residual Activity (%) vs pH

# F I G. 3
## (c)

Residual Activity (%) vs pH

○  Citric acid-Sodium citrate buffer

●  Imidazole-HCl buffer

□  Tris-HCl buffer

■  Sodium carbonate-Sodium
    hydrogencarbonate buffer

# F I G. 4

O  10 minutes  ,  □  1 days

●  2 hours  ,  ■  3 days

# F I G. 5

# F I G.6

| | | Molecular Weight |
|---|---|---|
| 1 : | Phosphorylase B | (92,500) |
| 2 : | Bovine Serum Albumin | (66,200) |
| 3 : | Ovalbumin | (45,000) |
| 4 : | Carbonic Anhydrase | (31,000) |
| 5 : | Soybean Trypsin Inhibitor | (21,500) |
| 6 : | Lysozyme | (14,400) |

# F I G.7

| | | Molecular Waight |
|---|---|---|
| 1 : | Bovine Serum Albumin | (67,000) |
| 2 : | Ovalbumin | (43,000) |
| 3 : | Chymotrypsinogen A | (25,000) |
| 4 : | Ribonuclease A | (13,700) |

0204284

# F I G. 8

Fraction Number

# F I G. 9

DISC
Electrophoresis

SDS
Electrophoresis

←B.P.B→